# DEMANDE DE BREVET EUROPEEN

(11) **EP 0 639 357 A1**
(43) Date de publication de la demande: **22.02.1995**
(21) Numéro de dépôt: 94420227.4
(22) Date de dépôt: 05.08.1994
(51) Int. Cl.: A61F 2/32

(54) **Cupule pour prothèse de hanche**

(30) Priorité: 06.08.1993 FR 9309923
(71) Demandeur: IMPLANTS ORTHOPEDIQUES TOUTES APPLICATIONS, S.A.R.L. dite:, F-42000 Saint-Etienne (FR)
(72) Inventeur: Deaux, Pierre Geoffroy, F-42000 Saint Etienne (FR)
(74) Mandataire: Perrier, Jean-Pierre

(57) **Abrégé**

Dans cette cupule, la bague de rétention (4) comporte un alésage cylindrique (21), dont le diamètre (d) est inférieur à celui de la tête fémorale, pour en assurer la rétention longitudinale, et plusieurs bossages arrondis (22), saillant radialement et de rayon maximal RB, tandis que la jupe (11), solidaire du noyau (3) ou de la cupule (2), comporte, une zone (9) avec des portées de contact pour les bossages (22) aptes à exercer sur le bossage correspondant une compression radiale déformant localement la bague et assurant une rétention plus élevée.

## Description

La cupule selon l'invention est du type associée à un noyau interne et à une bague de rétention de la tête fémorale d'une prothèse de la hanche.

Ce type de prothèse est utilisé lorsque la cavité cotyloïdenne est en bon état et ne justifie pas une remise en forme par le chirurgien. Il en résulte, qu'à la différence des cupules s'insérant après usinage de la cavité cotyloïdienne et ayant des dimensions correspondant à celle de l'outil de remise en forme, la cupule selon l'invention est choisie par le chirurgien en fonction des dimensions de la cavité cotyloïdienne. Cela implique qu'il procède à plusieurs essais au cours desquels il est amené à désolidariser la cupule de la pièce d'essai la portant ou de la pièce fémorale de la prothèse. A cette fin, la bague de rétention est fixée dans la cupule ou le noyau, de manière à pouvoir être démontée.

Dans les cupules actuelles, le mode de liaison mis en oeuvre est soit difficile à actionner, cas dans lequel le chirurgien rencontre des difficultés pour démonter la prothèse, soit procure, dans son état de démontage, une rétention nulle permettant une séparation aisée de la cupule et de la tête fémorale, mais permettant également, lors des manipulations de l'ensemble de la prothèse dans la salle d'opération que l'un des éléments de cette prothèse se désolidarise des autres et tombe au sol, avec les inconvénients qui en résultent, tant au niveau de la stérilité que de l'allongement du temps de pose.

La présente invention a pour but de fournir une cupule qui remédie à ces inconvénients et qui assure, d'une part, à l'état non bloqué, une rétention de la tête fémorale suffisante pour empêcher la séparation accidentelle des éléments de la prothèse, mais insuffisante pour en empêcher la séparation volontaire, et, d'autre part, à l'état bloqué, une liaison mécanique indémontable, même volontairement.

A cet effet, dans la cupule selon l'invention, la bague de rétention comporte :
- d'une part et au dessous de sa cavité hémisphérique, un alésage cylindrique dont le diamètre (D) est inférieur à celui (d) de la tête fémorale, d'une valeur de l'ordre de 0,2 mm, pour en assurer la rétention longitudinale,
- et, d'autre part, sur sa périphérie et au moins au dos de sa partie assurant la rétention de la tête fémorale, plusieurs bossages arrondis, saillant radialement et de rayon maximal (RB) par rapport à l'axe longitudinal de cette bague,

tandis que la jupe, solidaire du noyau ou de la cupule, comporte, en vis à vis de chaque bossage et sur un secteur angulaire de valeur au plus égal à 360°/n, où n correspond au nombre de bossages :
- d'une part, une portée d'engagement (PE), dont le rayon (RE) a une valeur égale à celui (RB) des bossages,
- et, d'autre part, une portée de verrouillage (PV) de rayon (RV) inférieur à celui (RB) des bossages, pour exercer sur le bossage correspondant une compression radiale déformant localement la bague et assurant une rétention plus élevée.

Grâce à cette structure, la prothèse peut être manipulée sans que la cupule puisse se séparer de la tête fémorale, tout en permettant au chirurgien d'effectuer cette séparation, s'il en a le besoin, et cela grâce à la rétention de faible valeur procurée la portée de rétention de la bague. Par ailleurs, lorsque les bossages de la bague sont amenées au voisinage des portées de verrouillage, ils sont soumis, par ces portées, à une contrainte radiale importante, déformant localement la bague, augmentant la rétention de la tête rotulienne et empêchant son extraction hors de la cavité interne de la cupule.

D'autres caractéristiques et avantages ressortiront de la description qui suit en référence au dessin schématique annexé, représentant à titre d'exemple non limitatif, une forme d'exécution de la cupule selon l'invention, dans le cas où elle comporte trois bossages.
Figures 1 et 2 sont des vues en coupe transversale de cette cupule lorsque, respectivement, ces éléments sont assemblés et désassemblés,
Figure 3 est une vue en plan par dessus de la bague,
Figure 4 est une vue partielle suivant III-III de figure 2, montrant plus en détails, et à échelle agrandie, la forme des portées de la jupe coopérant avec chaque bossage.

Dans ce dessin, la référence numérique 2 désigne la cupule proprement dite, 3 son noyau et 4 la bague de rétention.

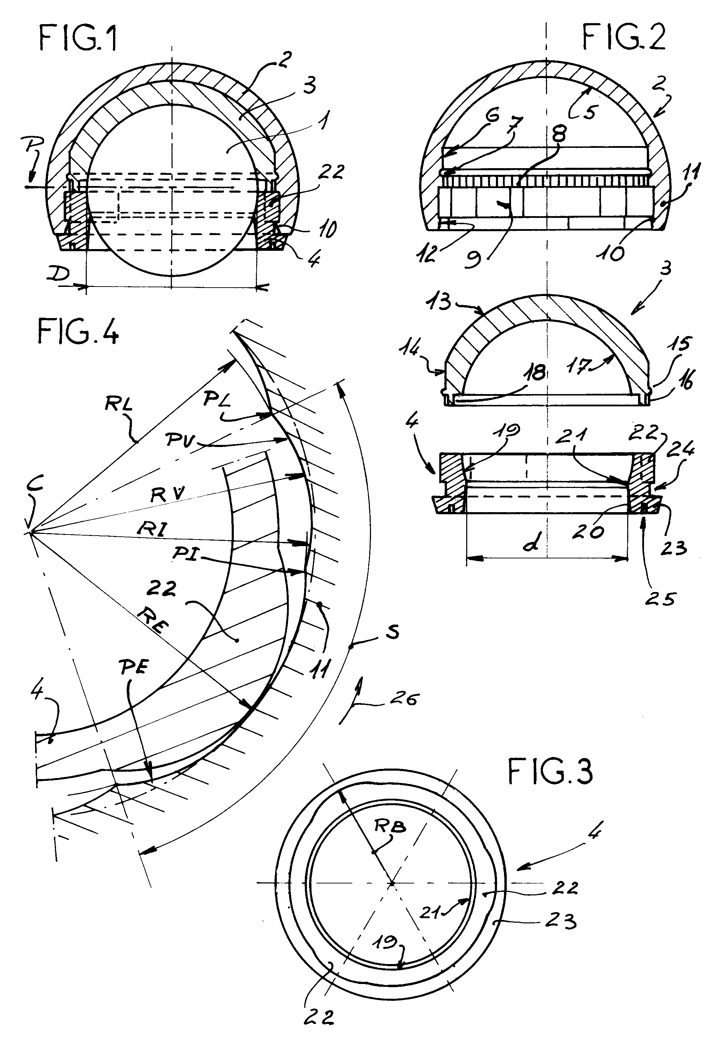

La cupule 2 est réalisée en métal et comporte, extérieurement, une forme hémisphérique correspondant à celle de la cavité cotyloïdienne. Intérieurement, elle comporte une cavité hémisphérique 5 se prolongeant, sur sa jupe 11, par une portée cylindrique 6, une gorge circulaire 7, des cannelures longitudinales 8, et une zone 9 comportant des portées de contact qui seront décrites, plus en détails, en référence à la figure 4. Enfin, la cupule comporte, au moins sur une partie de son embouchure circulaire, une ergot 10 précédé par un chanfrein 12.

Le noyau 3 est réalisé en matière synthétique présentant un faible coefficient de frottement et, par exemple en polyéthylène haute densité. Il comporte extérieurement une portée hémisphérique 13 apte à venir en appui sur la cavité 5 de la cupule, une surface cylindrique 14, une collerette 15, apte à s'encliqueter dans la gorge 7 de la cupule 2, et, enfin, des cannelures 16, aptes à coopérer avec celles 8 de la cupule. On notera que, lorsque le noyau 3 est engagé dans la cupule 2, il est calé en translation axiale par la collerette 15, encliquetée dans la gorge 7, et calé en rotation par les cannelures 16 et 8.

Intérieurement, le noyau 3 comporte une cavité hémisphérique 17 apte à venir en contact avec la tête hémisphérique 1 de l'élément fémoral de la prothèse de hanche. Cette cavité 17 est concave et tournée vers le bas et débouche dans un chambrage 18, de plus grande diamètre qu'elle, évitant tout risque de coincement avec la zone du plan diamétral de la tête fémorale.

La bague 4 est également réalisée en matière synthétique ayant un faible coefficient de frottement et comporte, en vis à vis de la cavité 17 du noyau 3, une cavité 19 en forme de segment sphérique dont la concavité est tournée vers le haut et est donc destinée à venir en contact avec la tête fémorale sphérique 1, au-dessous du plan diamétral de cette tête, représenté par le trait mixte P à la figure 1. Cette cavité se prolonge, vers le bas, par une portée de rétention 11 et par surface tronconique 20, évasée vers le bas, pour permettre les libres mouvements de la tige reliant la tête fémorale 1 au corps de la prothèse fémorale.

La portée de rétention 11 est constituée par un alésage cylindrique ayant un diamètre d qui est inférieur à celui D de la tête fémorale 1 d'une valeur de 0,2 mm.

Comme montré plus en détails à la figure 3, la bague de rétention 4 comporte plusieurs bossages 22 saillant radialement vers l'extérieur. Ces bossages arrondis s'étendent verticalement au moins sur la partie de la bague comportant la cavité 19 et la portée de rétention 11. Sur la figure 3, la distance maximale entre l'axe longitudinal de la bague et le point du bossage qui en est le plus éloigné, est représentée par le rayon RB. Chacun de ces bossages est destiné à coopérer avec des portées de contact ménagées dans la zone 9 de la jupe 11 de la cupule, mais pouvant également, dans une autre forme d'exécution, être ménagées sur une jupe prolongeant le noyau 3 et s'adossant sur la zone 9, alors cylindrique.

Comme le montre la figure 4, ces portées de contact s'étendent sur un secteur angulaire S qui est au plus égal à 360°/n, expression dans laquelle n correspond au nombre de bossages portés par la bague 3. Chaque secteur angulaire S comporte donc une portée d'engagement PE et une portée de verrouillage PV, séparées par un point intermédiaire PI, dont les dimensions radiales vont être définies par rapport à l'axe longitudinal de la cupule, référencé C à la figure 4.

La portée d'engagement PE a un rayon RE de valeur égale, au jeu fonctionnel près, à celle du rayon RB des bossages, de manière à ne pas influencer la rétention procurée par la portée 21 de la bague 4.

La portée de verrouillage PV a, dans sa partie venant en contact avec un bossage, un rayon RV inférieur à RB d'une valeur de l'ordre de 0,4 mm.

Le point intermédiaire PI a un rayon RI dont la valeur est inférieure à celle du rayon RB de l'ordre de 0,6 mm.

La portée PV se termine par un point de calage PL dont le rayon RL est inférieur à RB d'une valeur de l'ordre de 1 mm, de manière à constituer un point de blocage, s'opposant à la rotation de la bague.

Les valeurs indiquées ci-dessus sont théoriques car elles dépendent des tolérances de fabrication qui sont de plus ou moins 0,02 mm ou 0,05 mm selon que la jupe est en métal ou en matière synthétique.

Dans la forme d'exécution représentée, ces portées sont réalisées par usinage au moyen d'une fraise de diamètre constant, dont le centre est décalé radialement et angulairement d'une portée à une autre, mais il est évident qu'elles peuvent être réalisées par tout autre moyen, pourvu qu'elles forment un profil en came.

La figure 2 montre bien que la partie de la bague 4 comportant les bossages 22 est séparée d'une collerette 23 par une gorge 24 permettant son encliquetage sur l'ergot 10 de la cupule 2. La collerette 23 est munie de moyens, et par exemple de plusieurs puits 25, destinés à recevoir les ergots d'entraînement d'une clé d'actionnement.

La cupule selon l'invention est fournie au chirurgien en étant montée sur la tête sphérique de l'élément fémoral de la prothèse. Dans cette position d'assemblage, les bossages 22 de la bague 4 sont en contact avec les portées PE de la jupe 11 mais n'engendrent aucune compression radiale sur la bague 4, de manière que la rétention de la tête ne soit assurée que par la portée 21 de cette bague. Cette rétention, empêche la sortie accidentelle de la tête fémorale, mais tolère son extraction par un effort manuel axial.

Il ressort de ce qui précède que, dans cette position d'assemblage, les différents éléments de la cupule permettent la séparation volontaire de la tige fémorale et de la cupule, tout en formant un ensemble monolithique dont les éléments sont inséparables.

Lorsque le chirurgien a terminé ses travaux de positionnement, et qu'il souhaite fixer définitivement la cupule sur la tête fémorale, il lui suffit de faire pivoter la bague 4 dans le sens de la flèche 26 de la figure 4. Durant cette rotation d'une fraction de tour, chaque bossage 22 rencontre d'abord le point intermédiaire PI, qui forme un point dur indiquant au chirurgien qu'au-delà de cette position angulaire, il passe en position de verrouillage.

On notera que, bien que la bague soit soumise, lors du passage devant ces points durs, à des efforts de compression radiale très élevés, ceux-ci sont sans conséquence sur le matériau de la bague car ils sont instantanés et non continus.

En continuant le mouvement de rotation de la bague 4, le chirurgien amène les bossages 22 au contact des portées PV. Dans cette position, ces portées PV exercent, sur les bossages, des forces de compression radiale qui déforment localement la bague et assurent une rétention supérieure. Il en résulte que la rétention exercée par la bague sur la tête fémorale s'oppose à toute extraction longitudinale de cette tête et assure donc une solidarisation mécanique positive de la cupule avec la tige fémorale.

Le point PL informe le chirurgien de la fin de la course de verrouillage et l'incite à ne pas continuer la rotation. Cela évite que les bossages 22 passent dans la zone S suivante.

Il est évident, qu'en cas de réintervention, le chirurgien peut désolidariser la cupule de la tête fémorale en faisant pivoter la bague dans le sens inverse de la flèche 26.

Selon les applications et les dimensions de la cupule, le nombre des bossages peut varier de 3 à 8.

On notera que les crans, formés radialement dans la bague par les portées PV, délimitent, entre eux et avec la face de la tête fémorale, des couloirs qui, bien que de faible épaisseur, permettent au liquide de lubrification de l'articulation de circuler librement, ce qui améliore le fonctionnement de la prothèse et évite les conséquences néfastes des phénomènes de pompage par l'extension de la jambe.

## Revendications

1. Cupule pour prothèse de hanche du type associé à un noyau interne (3) en matière synthétique, comportant une cavité hémisphérique (17) apte à venir en contact avec la tête fémorale de la prothèse, et à une bague de rétention (4), également en matière synthétique, comportant une cavité (19) en forme de segment hémisphérique, apte à venir en contact avec la tête fémorale au-dessous du plan médian de celle-ci, **caractérisée en ce que** la bague de rétention (4) comporte :
- d'une part et au dessous de sa cavité hémisphérique (19), un alésage cylindrique (21) dont le diamètre (D) est inférieur à celui (d) de la tête fémorale (1), d'une valeur de l'ordre de 0,2 mm, pour en assurer la rétention longitudinale,
- et, d'autre part, sur sa périphérie et au moins au dos de sa partie (19-21) assurant la rétention de la tête fémorale, plusieurs bossages arrondis (22), saillant radialement et de rayon maximal RB par rapport à l'axe longitudinal de cette bague,
tandis que la jupe (11), solidaire du noyau (3) ou de la cupule (2), comporte, en vis à vis de chaque bossage (22) et sur un secteur angulaire (S) de valeur au plus égal à 360°/n, où n correspond au nombre de bossages:
- d'une part, une portée d'engagement (PE), dont le rayon (RE) a une valeur égale à celui (RB) des bossages,
- et, d'autre part, une portée de verrouillage (PV) de rayon (RV) inférieur à celui (RB) des bossages, pour exercer sur le bossage correspondant une compression radiale déformant localement la bague et assurant une rétention plus élevée.

2. Cupule selon la revendication 1, **caractérisée en ce que** la bague (4) est immobilisée en translation axiale sur la cupule (2) par encliquetage d'une gorge (24), dont elle est munie, sur un ergot périphérique interne (10) de la cupule (2).

3. Cupule selon l'une quelconque des revendications 1 à 2, **caractérisée en ce que** la portée d'engagement (PE) a un rayon (RE) de valeur égale à celle du rayon (RB) des bossages.

4. Cupule selon l'une quelconque des revendications 1 à 3, **caractérisée en ce que** la portée de verrouillage (PV) a, dans sa partie venant en contact avec un bossage, un rayon (RV) de valeur inférieure, de l'ordre de 0,4 mm, à la valeur du rayon (RB) des bossages.

5. Cupule selon l'une quelconque des revendications 1 à 4, **caractérisée en ce qu'**un point dur anti-retour (PI) est intercalé entre la portée d'engagement (PE) et la portée de verrouillage (PV).

6. Cupule selon la revendication 5, **caractérisée en ce que** le point (PI) a un rayon (RI) dont la valeur est inférieure, d'au moins 0,6 mm, à celle du rayon (RB) des bossages (22).

7. Cupule selon l'une quelconque des revendications 1 à 6, **caractérisée en ce que** la portée de verrouillage (PV) est prolongée, vers l'aval dans le sens du verrouillage, par un point (PL), de calage en rotation et dont le rayon (RL) a une valeur inférieure , de l'ordre de 1 mm, à celle du rayon (RB) de bossages.

8. Cupule selon l'une quelconque des revendications 1 à 7, **caractérisée en ce que** le noyau (3) est calé en rotation dans la cupule (2) par coopération de cannelures (16), ménagées à l'extérieur de sa jupe, avec des cannelures complémentaires (8), ménagées dans la cupule (2), et est calé en translation axiale par encliquetage d'une nervure périphérique externe (15) dans une gorge interne (7) de cette cupule.
